# EUROPEAN PATENT APPLICATION

(11) **EP 2 439 536 A1**
(43) Date of publication of application: **11.04.2012**
(21) Application number: 10185508.8
(22) Date of filing: 01.10.2010
(51) Int. Cl.: G01N 33/86, G06F 19/00

(54) **New classification method for spectral data**

(71) Applicant: Nederlandse Organisatie voor toegepast- natuurwetenschappelijk onderzoek TNO, 2628 VK Delft (NL)
(72) Inventor: Paauw, Armand, 2628 VK Delft (NL); Parchen, René, 2628 VK Delft (NL)
(74) Representative: Jansen, Cornelis Marinus

(57) **Abstract**

The present invention relates to a new method for classification of spectral data comprising:
a. analyzing at least two samples belonging to at least one cluster through recording of a spectrum;
b. for each spectrum determining the peaks and the spectral value at which they occur;
c. calculating the probability (p) of occurrence for each peak for every cluster and from this the odds ratio p/(1-p) for each peak ;
d. preparing a spectrum from a sample to be classified with the same technique as in step a);
e. determine the peaks in the spectrum obtained in step d);
f. calculate the likelihood of identity for each cluster by multiplying per cluster the odds ratio in said cluster for each peak found in the spectrum of step d); and
g. assign the sample tested in step d) to the cluster that provides the
largest number as a result of step f).

The invention also comprises a system for performing such a method and the use of such a system for classification of spectral data.

## Description

### The invention

The present invention relates to methods for data processing, more specifically for data processing of data derived from spectrographic analyses, such as mass spectrometry, MALDI, Raman spectrometry, and the like, in order to classify an unknown sample as belonging to a cluster of already identified samples.

### BACKGROUND

Nowadays, spectrographic methods are frequently used to analyse biomaterials. Whereas initially spectrometry was most heavily used in the fields of chemistry, and for the detection of (single) reaction compounds, it has increasingly become used in the biosciences for the analysis of biological samples.

In these analyses it can be the goal to analyse whether or not a known substance is present in the sample - as is the case in the detection of biomolecules such as proteins or polysaccharides in a sample -, but it can also be used to monitor biological processes. In biological samples in most cases many components, each giving their own spectral peaks, are contained within the sample. For the interpretation of these complex spectra additional aids need to be developed. Some existing methods for analysis include pattern recognition techniques and visual interpretation of spectra. Many techniques use some form of statistical analysis, such as principal component analysis, and similar multivariate analysis methods.

Spectrometry is also used for the analysis of complete organisms, such as bacteria and viruses. In such a case, it is not so much of importance which exact individual chemical compounds are reflected in the spectrum, but the goal of this analysis is much more to identify and classify the microorganism(s) in the sample. It has been recognized in the meantime that different microorganisms yield markedly different spectral data (irrespective whether the measurement is through a mass spectrometry, such as MALDI, Raman spectrometry, infrared spectrometry, or any other spectrometrical method). Many applications have hitherto been proposed for measuring and classifying microorganisms using spectral data (e.g. WO 2010/062354, WO 2010/062351, WO 2007/056568, CN 2009/1202963, WO 2005/060380, US 2004/234952, WO 2004/015136, WO 01/79523, JP 2028772).

In the mean time also commercial software applications for analysis and classification of these type of spectra have been developed, such as the SARAMIS™ system of Anagnos Tec. (EP 1253622) and Bruker Biotyper™ software (GB2467636). Further calculation models have been described in WO 02/096540, US2008/0318213 and US 7,555,393. These methods classify the spectra by comparing the individual spectrum to be classified with each and every spectrum of a reference set and classifying the sampled spectrum in the class that is established by the spectrum that most looks alike the analysed spectrum. The above mentioned statistical methods can be applied for calculating the measure of identity.

The problem that remains in these classification methods is that more weight is given to the presence of peaks in the spectrum and not to the absence of peaks in the spectrum, and further these systems do not or not adequately compensate for the variability that can be seen in biological samples from individual members of the same class.

### SUMMARY OF THE INVENTION

The present inventors now have devised a method that is able to overcome the above-mentioned difficulties. Therefore, the invention comprises a method for classification of spectral data by:
a) analyzing at least two samples belonging to at least one cluster through recording of a spectrum, where this spectrum can be selected from the group consisting of a MALDI-MS spectrum, a MALDI-TOF MS spectrum, a Raman spectrum, an FT-IR spectrum, a near-infrared (NIR) spectrumor a frequency spectrum, preferably a MALDI TOF MS spectrum;
b) for each spectrum determining the peaks and the spectral value at which they occur;
c) calculating the probability (p) of occurrence for each peak for every cluster and from this the odds ratio p/(1-p) for each peak;
d) preparing a spectrum from a sample to be classified with the same technique as in step a);
e) determine the peaks in the spectrum obtained in step d);
f) calculate the likelihood of identity for each cluster by multiplying per cluster the odds ratio in said cluster for each peak found in the spectrum of step d); and
g) assign the sample tested in step d) to the cluster that provides the largest number as a result of step f).

In another embodiment, the sample is a biological sample, preferably comprising a microorganism, such as a bacterium or a virus.

In a further embodiment a peak in the spectrum obtained in step d) is equivalent with a peak in the spectrum obtained in step b) if it occurs at the same spectral value, or at a spectral value that lies within the range of 98-102% of the spectral value of the peak detected in step b. In an other preferred embodiment the amplitude of the peak is taken account of in the calculation of the odds ratio the peak values per spectrum information by correction with a weighing factor. In a further preferred embodiment the number of samples for each cluster in step a) is at least 2, more preferably at least 5, most preferably at least 10.

The invention also relates to a system for the classification of spectral data, comprising a spectrometer, a database for the storage of spectral data, a database for storage of relevant information on the spectra or the samples from which the spectra are recorded, and a processor with instructions to carry out the calculations as described in any of the previous claims, wherein all these are connected to each other. In said system the spectrometer preferably is a MALDI-TOF MS spectrometer.

Also comprised in the invention is the use of a system according to the invention for the classification of biological material, preferably micro-organisms, more preferably bacteria or viruses.

### DESCRIPTION OF THE FIGURES

Fig. 1 MLVA clustering of 129 *Brucella* isolates. Each color represents a cluster. Cluster is defined as a group (or single isolate) of isolates of which the genetic relationships are >50% according UPGMA clustering.
Fig. 2. Results of MALDI-MS algorithm compared to MLVA results. Y-axis species according MLVA genotyping with biovar number. X-axis Measurements; from each isolate 8 MS-spectra were generated. Open circle results according MALDI-MS algorithm of the invention. Closed circle results according MLVA.

### DETAILED DESCRIPTION

Every microorganism strain produces a spectrum that can be regarded as a fingerprint pattern with specific signals at strain, species, genera and family level. These specific signals can be used for automated identification of unknown strains.

Requirements for modern methods suitable for microorganism identification and characterization are robustness, simple handling, low costs, speed and high-throughput capability. Therefore, spectrometry techniques, such as mass spectrometry may offer significant advantages over classical technologies e.g., polymerase chain reaction, sequencing, electrophoretic separation of nucleic acid fragments.

A commercial system for identification of microorganisms on basis of MALDI TOF mass spectrometry is available from AnagnosTec under the trade name SARAMIS™. In this system a number of reference spectra (so-called SuperSpectra™) is provided against which a sample spectrum is compared and as result of that comparison the sample spectrum (and thus the microorganism) is assigned to the cluster which is represented by this spectrum. The disadvantage of this system is that the reference spectra are fixed, i.e. they represent a number of clusters (which can be genera, species, or any other classification), and it is not envisaged to group the spectra in any other manner (such as pathogenic/non-pathogenic; endemic/non-endemic, etc). A second disadvantage is that the analysis is made by a comparison of the sample spectrum against each individual spectrum of the reference database, without accounting for the variation within the cluster from which the reference spectrum is derived. A third disadvantage is that this system is not a self-learning system. Each addition to the reference spectra will be a closely monitored exercition, and testing a new sample does not automatically mean that the reference set is adapted to accommodate a better analysis after including said new sample in the reference database.

In the present method these disadvantages have been overcome, while using a simple and reliable analysis method to determine the identity of a sample spectrum to a set of reference spectra. For this system, as for the other systems that are available, first a set of reference spectra has to be established. This is performed by taking individual spectra of biological samples that include characteristic examples of microorganisms that are expected to occur in samples that are to be tested. For each spectrum it is determined which peaks are present in the spectrum. It is not specifically critical in which way the peaks are determined, as long as the same method is applied for all spectra to be measured. Many algorithms for peak detection are available, and it is for the current invention not specifically critical which one is used Mostly, the peak detection process comprises three stages: smoothing, baseline correction, and peak picking. For each stage different algorithms are available. Examples of algorithms for smoothing are continuous wavelet transformation, moving average filter, or the Savitzky-Golay filter. Baseline correction can be done with algorithms that determine the monotone minimum, Loess, or linear interpolation. Examples of algorithms for peak picking are based on signal to noise ration, local maximum or peak width. [Comparison of public peak detection algorithms for MALDI mass spectrometry data analysis. Yang C, He Z, Yu W. BMC Bioinformatics. 2009 Jan 6;10:4.]

After this it is possible to divide the spectra that have been measured up into a number of clusters. These clusters can concur with a classical taxonomical classification of the microorganisms, but the number of clusters and the content of the individual spectra therein is free of choice. Of course, the more mixed and heterogeneous the clusters are, the more difficult it will prove to achieve a reliable classification. Also, to increase the reliability of correct classification with the present system, it is advisable to include a minimal number of spectra per cluster, e.g. 10, 50, 100 or even more.

Then for each cluster and each peak the percentage of individual spectra within that cluster that contain said peak is calculated. It is a possibility to also discount for the amplitude of the peak in this system. The amplitude of the peak could help in the discrimination between different clusters. For this, the relative amplitude of the peak in respect of the total amplitude of the spectrum could be used as a weighing factor. In such a case not only a value of 0 (peak absent) or 1 (peak present) could be entered, but for example also values of 0.5 (peak of lesser amplitude), 1 (peak of 'normal' amplitude) and 2 (peak of higher amplitude) can be entered (other values than the mentioned ones can easily well be applied). Of course, such a determination of the peak occurrence can bring the total 'percentage' to more than 100, but this is no problem for the further calculation.

From this 'total percentage' the probability of occurrence (p) is calculated by dividing the percentage of one cluster by the total percentages of the other clusters

The odds ratio (for each peak for each cluster) is then calculated as p divided by (1-p): p·(1-p)⁻¹.

This can be illustrated by the following example:

Suppose spectra are obtained for three clusters of microorganisms X, Y and Z, with in each cluster sufficient spectra. In total 5 peaks in the spectra are visible and the percentages of occurrence in this group are according to Table 1:
)

**Table 1: Example of percentage occurrence of peaks in spectra of three clusters (fictious data**

| peak | cluster X | Cluster Y | Cluster Z |
|---|---|---|---|
| A | 1 | 95 | 1 |
| B | 1 | 50 | 90 |
| C | 10 | 50 | 20 |
| D | 5 | 5 | 25 |
| E | 99.9 | 30 | 99.9 |
| F | 90 | 10 | 80 |

From this data the probability of occurrence (p) for each peak in each cluster is calculated by taking the percentage in that cluster and dividing it by the total sum of percentages of all clusters. In this case the first value p of peak A in cluster X is calculated as 1 divided by 1 + 95 + 1 = 1/97 = 0.010309.

The table of p values then will be:

**Table 2: p-values per peak per cluster of the data of Table 1.**

| peak | cluster X | cluster Y | cluster Z |
|---|---|---|---|
| A | 0.010309 | 0.979381443 | 0.010309 |
| B | 0.007092 | 0.354609929 | 0.638298 |
| C | 0.125 | 0.625 | 0.25 |
| D | 0.142857 | 0.142857143 | 0.714286 |
| E | 0.434726 | 0.130548303 | 0.434726 |
| F | 0.5 | 0.055555556 | 0.444444 |

The odds ratio is determined as p·(1-p)⁻¹ and thus will give the following table for these data:

**Table 3: Odds ratio calculated from the p-values in Table 2.**

| peak | cluster X | cluster Y | cluster Z |
|---|---|---|---|
| A | **0.010417** | **47.5** | **0.010417** |
| B | **0.007143** | **0.549451** | **1.764706** |
| C | **0.142857** | **1.666667** | **0.333333** |
| D | **0.166667** | **0.166667** | **2.5** |
| E | **0.769053** | **0.15015** | **0.769053** |
| F | **1** | **0.058824** | **0.8** |

Please note that it is preferred to always enter a positive number (i.e. not zero) in the table for the percentage occurrence of a peak in the spectra of one cluster, even if there are zero occurrences. This, because otherwise also an odds ratio of 0 would result, which may be undesired in the light of further calculations as discussed below

It will be apparent that in this system it would be possible to implement a different definition of the clusters, involving e.g. grouping or splitting the existing clusters, or which new clustering could even include a totally different classification paradigm. It will be easy to recalculate the odds ratios according to such a new set of clusters.

After having compiled the odds ratio table, a spectrum of a sample to be tested is made and the peaks therein are determined according to the method described above, although in this case no weighing factor for the amplitude is necessary. Then, this spectrum is represented by a list of the spectral values at which the peaks occur. This list is held along the odds ratio table and per cluster for every peak that has been found in the spectrum of the new sample the corresponding odds ratio in that cluster is taken. After all peaks have been checked, the odds ratios of the cluster belonging to these peaks are multiplied, this gives the result value for said cluster.For each cluster the result value is calculated and the sample is determined to belong to the cluster with the highest result value.

Instead of multiplication any other mathematical operation can be used. The method that gives optimal results depends on the correlation between peaks. If the occurrence of peaks is mutually strongly correlated, multiplication (or simple addition) of the odds ratio's will already provide good results. If the occurrence of peaks is not strongly correlated, the odds ratio's belonging to the peaks that occur in the spectrum can be regarded as new variables that characterize the spectrum. Several techniques from statistical analysis, such as principal component analysis, and similar multivariate analysis methods can be used to formulate a measure for the similarity with the different clusters.

To enlighten this step in respect of the previous example data, assume that the new spectrum would yield the peaks A, B and C. Then for cluster X the cluster result value by multiplication is 0.010417 times 0.007143 times 0.142857, which would yield 0.000013 (13.10-⁶). For clusters Y and Z, the final result would be 43.49 and 0.006, respectively. If the numbers are added the results would be 0.16, 49.71 and 2.11. In this case thus both types of calculations yield reliable results with an identical outcome. On basis of these results, the spectrum will be classified as belonging to cluster Y. It is clear that the likelihood that the sample spectrum belongs to one of the clusters is higher as the result value of a cluster is higher, and conversely lowers, if the result value is lower. The values, however, also would serve to give a measure of relatedness or rank, in which the clusters with higher values are more related to the sample spectrum than clusters with lower values.

It is also envisaged that a conclusion can be drawn that the sample spectrum does not fall within one of the clusters. Such a conclusion can be drawn if either all the result values are very low, and/or if two or more clusters yield top result values that come very close (irrespective of their absolute value). Nevertheless, in such a case, it will still be possible to rank the clusters with respect to their similarity to the sample spectrum.

A further advantage of the present method is that the system is self-learning. This can be accommodated by adding the sample spectrum (or more sample spectra) to one of the clusters and to recalculate the odds ratio table for this new set of clusters, taking into account the addition of the one (or more) sample spectra.

The present method can be applied to all sorts of spectra, such as mass spectra, emission spectra, absorption spectra, frequency spectra and the like. It is submitted that the method can also be used for classifying any sample spectrum into a predefined cluster on the basis of the similarity of the spectra (more specifically, the similarity of the peaks in the spectra). The invention, however, is especially useful in the classification of microorganisms where the spectra are obtained from Raman spectrometry or mass spectrometry. In the latter case, the spectra preferably are MALDI-TOF spectra.

The recording of the spectrum can be achieved by the instrument that is suited for such a measurement, such as a mass spectrometer or a Raman spectrometer. The processing means, which will typically be a computer which, in operation, executes a computer program, is also part of the present invention. The computer may be a personal computer, or any other type of processing device, such as a single processor or multiprocessor system The program may be stored in a storage medium, such as, e.g., a floppy disk or CD-ROM which is read by a medium drive device such as, e.g., a floppy disk drive or a CD ROM drive. Alternatively, the program is stored in a storage medium forming part of the computer, such as e.g., a hard disk or other memory devices.

The computer program in operation executes computer executable software code for analysis of the signal obtained from the spectrometer and for classification of the microorganism according to the analysis method and/or classification method as described above.

When the new spectrum of a sample is classified within a cluster of spectra that were already in the database, the identity of the sample to the cluster was established by virtue of spectroscopic similarity. This identity is then used to look up in an information database (which can form part of the spectral database or may be a completely separate database) the available information about that cluster, which was then presented to the user. The information database contains information about the clusters of which spectra are present in the spectral database, which information in the case of microorganisms can be, e.g. taxonomical classification, antimicrobial agent susceptibility, virulence, known complications, etc.

If the new spectrum can not be classified unequivocally it is classified as an unknown cluster. This cluster receives a unique code-name, and the user is prompted, e.g. visually and/or audible and/or by an electronic message, to enter available information about this sample into the information database. This information may comprise e.g. the results of other techniques for identification, which in case of microorganisms may be phenotypic or genotypic and at any taxonomic level, an antibiogram, date of isolation, (patient) material from which the microorganism was isolated, and clinical complications caused by the infection. At all times it is possible for the user to update the information database when new information about a cluster becomes available, from whatever source. This may include information obtained by electronically linking and comparing information databases at regular intervals. If the new spectrum was identified as belonging to a clustered which relates to microbial strain Q, all information about strain Q, which is stored in the information database becomes available and information about the sample from which the new spectrum was obtained is added to the information database.

Preferably, all new spectra are immediately added to the cluster of spectra which results from the classification. In this way, they are immediately available to aid in identification of subsequent new samples. Of course, addition of new spectra to the clusters necessitates the re-calculation of the odds table.

The dataset of spectra available to serve as reference for newly measured spectra may continually and automatically be expanded with another measured spectrum. It is noted that the above embodiment for automated generation and automated updating of a database and its use in analyzing new spectra, is given only by way of example. It will be clear to those skilled in the art that choices for criteria that are applied and signal analysis methods that are used, can be replaced by alternatives.

The spectral database of the instrument of the invention thus comprises spectra from the spectrometer which have been classified into clusters according to known or new classification models. The database is automatically adapted or extended by the incorporation of new spectra. It may comprise microbial spectra of subspecies specificity.

The spectral database preferably comprises, next to the spectral data, information on the spectrum, such as time and date of recording, sample identification, spectrum identification, spectrometric parameters used in the recording of the spectrum (such as filters, light/energy source and the like) and/or operator identification.

More information about the spectra in the spectral database may be obtained from the information database, which is part of the spectral database or to which the spectral database may optionally be connected.

Connections between the first spectral database and other databases may be established by any means of data transfer and suitable data-transfer protocols, including but not limited to wireless data transfer, intranet systems, internet, the use of portable data storage devices such as computer diskettes and compact disks. The information database according to the invention comprises specific information on the cluster and/or each individual sample therein, comprising but not limited to sample identification, spectrum identification, time and date of sampling, peculiarities of the sample, such as addition of buffers, any pre-recording treatment of the sample (such as washing, filtering, etc.). If the sample contains biological material, the source and nature of the biological material may be part of the additional information. When the sample is a microorganism, the information database may contain information on prevalence, virulence, clinical complications, antimicrobial agent susceptibility, which data becomes instantly available. Moreover such information may be updated with the sample and/or patient information of the new sample of which the spectrum was obtained. Such information includes, but is not limited to, the time and date the patient material was obtained, the type of patient material used, the clinical condition of the patient and or the changes in the clinical condition of the patient, treatments, including the treatment for the infection and the effect thereof, diagnostic procedures that the patient has undergone, whether or not the infection has manifested itself after the patient was admitted to a hospital, antimicrobial agent susceptibility profile of the microbial strain, whether the microbial strain is or has been involved in an outbreak, virulence of the microbial strain, whether the isolated microbial strain is locally endemic (pointing to persistent source(s) of contamination), wards and departments where a patient has stayed or has been examined, taxonomic classification by other methods (including classification at genus, species and/or sub-species level), such as for instance 16S RNA sequencing Multi Locus Variable tandem repeat Analysis (MLVA), Multi Locus Sequence Typing (MLST) and other methods.

In this way, the invention allows for sub-species level specific information to be obtained from a microbial strain. It also provides rapid access to useful clinical data such as best course of treatment, know complications of an infection with the particular strain and e.g. virulence of the microorganism. At the same time it provides information regarding earlier cases of infection with the same microorganism. This allows for the rapid determination of a source from which the microorganism is spread, such as for instance a non sterile medical device, which requires additional measures to be taken, or a foodstuff.

In another preferred embodiment, the spectral database or algorithm based on this spectral database, and the information database are combined in one single database.

In another aspect, the instrument used for the methods of the invention further comprises a second spectral database and a second information database. This second spectral database may comprise spectra which are not present in the first spectral database and the second information database may comprise additional information about the microorganisms in the second spectral database.

The instruments for measuring and/or the databases(s) may be part of a network, such as a local, regional or global area network. The term "network", refers to two or more computers or processing systems which are connected in such a way that messages and information may be transmitted between the computers. In such computer networks, typically one or more computers operate as a "server", a computer with large storage devices such as hard disk drives and communication hardware to operate peripheral devices such as printers or moderns. Other computers, termed "workstations" or "clients", provide a user interface so that users of computer networks may access the network resources, such as shared data files, common peripheral devices, and inter-workstation communication. Users activate computer programs or network resources to create "processes" which include both the general operation of the computer program along with specific operating characteristics determined by input variables and its environment. The network will comprise at least one server and at least one, and typically several workstations. Server and workstations are connected by a communication line, which may be an ethernet cable or another suitable device, such as a wireless connection. The network may also include several shared peripheral devices. In one embodiment of the invention, the spectrometer is a remote facility which is connected to the computer by a server.

A local, regional or global network of spectrometer and databases(s) may be suitably used to monitor geographical presence and changes therein of microbial strains. It may automatically issue an alert if an unusual change in geographical presence has been detected. Unusual changes include, but are not limited to the prevalence of a new strain. Such network also allows for obtaining retrospectively epidemiological data without the requirement to do additional testing. In addition, it is possible to prospectively assemble epidemiological data..

The system of the invention can further comprise a signal which is or can be made visible or audible output in one or more of the following categories:
- prompting the user that the spectrum of the sample of interest is already present in the first spectral database;
- prompting the user to apply other means of characterization;
- prompting the user to enter information in an information database;
- suggesting suitable antimicrobial therapy;
- alerting the user to a change in antimicrobial agent susceptibility profile;
- alerting the user of a persisting contamination;
- alerting the user when an unusual change in geographical presence occurs is also part of the invention..

Although the present description is written with a focus on the classification of microorganisms from biological or environmental samples, the spectral analysis method of the present invention may also be used in other applications, such as voice recognition systems, spoken instruction recognitions systems, detection of chemical or biological compounds in complex samples, and the like

### EXAMPLE

The genus *Brucella* contains highly infectious species, able to cause infections in a wide variety of mammals. Until recently, six species where assigned to the *Brucella* genus, where some species contain different biovars: *Brucella abortus* (7 biovars), *Brucella melitensis* (3 biovars), *Brucella suis* (5 biovars), *Brucella ovis, Brucella canis,* and *Brucella neotomae.* Four new species have been described recently. Three of these species were isolated from 'wild' mammals and sea mammals: *Brucella ceti, Brucella pinnipedialis,* and *Brucella microti.* Finally, a new species was cultured from a breast implant infection and named *Brucella inopinata* (Scholtz, H.C. et al., 2010, Int J Syst Evol Microbiol. 60:801-808).

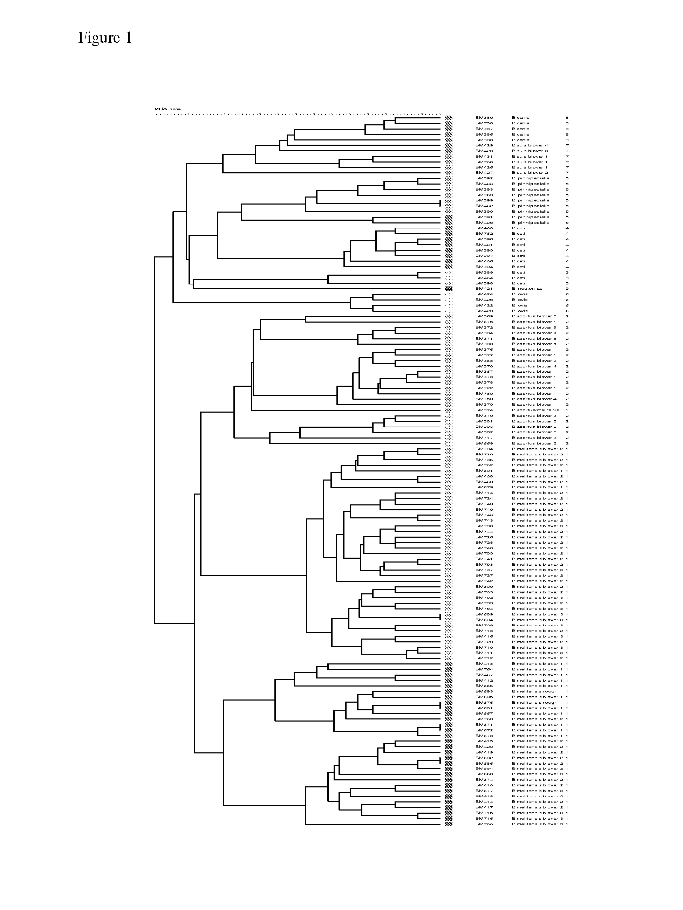

To improve the discriminatory power of MALDI-TOF MS based identification of *Brucella* isolates, a new approach was developed. First, Multi Locus Variable number tandem repeat Analysis (MLVA) was performed on 129 samples. MLVA clustering divided the isolates into 16 clusters, based on 50% genetic relatedness threshold using an UPMGA clustering (Figure 1). Nomenclature of the clusters is based on the species identification performed by comparing the MLVA results against the public *Brucella* MLVA database. Next, the MALDI-TOF MS spectrum of each of the 129 isolates was recorded in eightfold (sometimes sevenfold), yielding 1028 MS-spectra. These spectra were pre-processed, using standard algorithms. Pre-processing steps that were applied comprised of smoothing, resampling, base-line subtraction and normalizing. Finally, at each mass bin, the presence or absence of a peak was determined thus converting the spectra to peak lists.

Based on the MLVA based clustering, each spectrum was assigned to its representative cluster. Subsequently, for each mass bin, the percentage that a peak was present or absent was determined for each cluster and from that the odds-ratio for each mass bin in each cluster was calculated. Since there is always a finite probability that a peak occurs at a position that is previously defined as a peak position in a specific cluster the minimal probability was set on 1.10⁻¹⁰.

Once the odds-ratio table was constructed, it could be used to evaluate the similarity of an unknown test spectrum to the different clusters.

For this purpose the unknown test spectrum had to be be converted to a peak list, using the identical pre-processing steps as those used during the construction of the odds-ratio table. The product of the odds-ratios at each peak location in the test spectrum yielded a measure for the similarity of the test spectrum to each of the clusters. The spectrum was assigned to the clusters that yielded the highest value of the odds-ratio product.

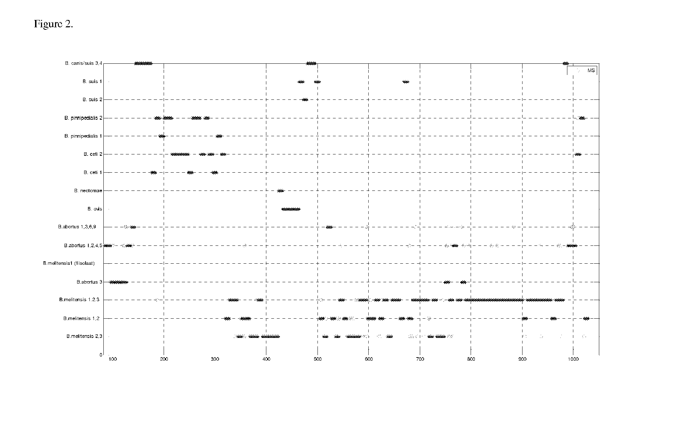

Figure 2 compares the classification results based on the MALDI odd-ratio approach (green open cirles) to the MLVA clustering (blue dots). If the MALDI odd-ratio approach and the MLVA assign the sample to the same cluster, the green open circle and the blue dots will plot at the same position. If the methods yield conflicting classifications, the symbols are plotted at a different positions.

According to figure 2 the number of corresponding classifications is much larger than the number of conflicting classifications. Those discrepancies that do occur, occur only for closely related species, illustrating the excellent performance of the proposed method.

## Claims

1. A method for classification of spectral data comprising:
a. analyzing at least two samples belonging to at least one cluster through recording of a spectrum;
b. for each spectrum determining the peaks and the spectral value at which they occur;
c. calculating the probability (p) of occurrence for each peak for every cluster and from this the odds ratio p/(1-p) for each peak ;
d. preparing a spectrum from a sample to be classified with the same technique as in step a);
e. determine the peaks in the spectrum obtained in step d);
f. calculate the likelihood of identity for each cluster by multiplying per cluster the odds ratio in said cluster for each peak found in the spectrum of step d); and
g. assign the sample tested in step d) to the cluster that provides the largest number as a result of step f).

2. Method according to claim 1, wherein the spectrum is selected from the group consisting of a MALDI-MS spectrum, a MALDI-TOF-MS spectrum, a Raman spectrum, an FT-IR spectrum, a near-infrared (NIR) spectrum and a frequency spectrum.

3. Method according to claim 1 or claim 2, wherein the sample is a biological sample, preferably wherein said biological sample comprises a microorganism.

4. Method according to claim 3, wherein the spectrum is a MALDI-TOF-MS spectrum.

5. Method according to any of the previous claims, wherein a peak in the spectrum obtained in step d) is equivalent with a peak in the spectrum obtained in step b) if it occurs at the same spectral value, or at a spectral value that lies within the range of 98-102% of the spectral value of the peak detected in step b

6. Method according to any of the previous claims where in the calculation of the odds ratio the peak values per spectrum information on the amplitude of the peak is taking account of by correction with a weighing factor.

7. Method according to any of the previous claims, wherein the number of samples for each cluster in step a) is at least 3, more preferably at least 5, most preferably at least 10.

8. System for the classification of spectral data, comprising a spectrometer, a database for the storage of spectral data, a database for storage of relevant information on the spectra or the samples from which the spectra are recorded, and a processor with instructions to carry out the calculations as described in any of the previous claims, wherein all these are connected to each other.

9. System according to claim 8, wherein the spectrometer is a MALDI-TOF MS spectrometer.

10. Use of a system according to claim 8 or 9 for the classification of biological material, preferably micro-organisms, more preferably bacteria or viruses.
